(19) Europäisches Patentamt
European Patent Office
Office européen des brevets

(11) Veröffentlichungsnummer: **0 010 587**
**B1**

(12) # EUROPÄISCHE PATENTSCHRIFT

(45) Veröffentlichungstag der Patentschrift: **01.06.83**

(21) Anmeldenummer: **79103160.2**

(22) Anmeldetag: **27.08.79**

(51) Int. Cl.³: **C 07 C 103/52,**
C 07 C 69/96,
C 07 C 125/06

(54) **Adamantylalkyloxycarbonylderivate, deren Herstellung und Verwendung zur Darstellung von Peptiden.**

(30) Priorität: **07.09.78 LU 80207**

(43) Veröffentlichungstag der Anmeldung:
**14.05.80 Patentblatt 80/10**

(45) Bekanntmachung des Hinweises auf die Patenterteilung:
**01.06.83 Patentblatt 83/22**

(84) Benannte Vertragsstaaten:
**AT BE CH DE FR GB IT NL SE**

(56) Entgegenhaltungen:
**DE - A - 1 924 802**
**DE - A - 1 930 330**
**DE - A - 2 063 087**
**DE - A - 2 807 403**
**DE - B - 1 443 949**
**GB - A - 1 106 272**

(73) Patentinhaber: **Kalbacher, Hubert**
**Hefelestrasse 15**
**D-7407 Rottenburg (DE)**

(73) Patentinhaber: **Voelter, Wolfgang, Prof. Dr.**
**Panoramastrasse 71**
**D-7400 Tübingen (DE)**

(72) Erfinder: **Kalbacher, Hubert**
**Hefelestrasse 15**
**D-7407 Rottenburg (DE)**
Erfinder: **Voelter, Wolfgang, Prof. Dr.**
**Panoramastrasse 71**
**D-7400 Tübingen (DE)**

(74) Vertreter: **Kraus, Walter, Dr. et al,**
**Patentanwälte Dres. Kraus & Weisert**
**Irmgardstrasse 15**
**D-8000 München 71 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 010 587 B1

# Adamantylalkyloxycarbonylderivate, deren Herstellung und Verwendung zur Darstellung von Peptiden

## Beschreibung

Die Erfindung betrifft neue substituierte Kohlensäureester und Urethane, Verfahren zur ihrer Herstellung und deren Verwendung.

Es sind zahlreiche Schutzgruppen bekannt, die bei Umsetzung von primären und sekundären Aminen, insbesondere Aminosäuren, biogenen Aminen und Aminozuckern Verwendung finden. Eine neuere zusammenfassende Darstellung findet sich z.B. in den beiden Büchern von E. Wünsch in E. Müller (Herausgeber): Houben-Weyl, Methoden der Organischen Chemie 15/1, Georg Thieme Verlag Stuttgart 1974 and H. D. Jakubke und H. Jeschkeit, Aminosäuren-Peptide-Proteine, 2. Auflage, Akademie-Verlag Berlin 1973.

Obwohl die bekannten Aminoschutzgruppen von Urethantyp (Boc, Z) einen weiten Teil der Anforderungen von eindeutig verlaufenden Syntheseaufbauprinzipien abdecken, ist es in vielen Fällen wüschenswert, Schutzgruppen einzusetzen, die unter sehr milden acidolytischen Bedingungen abspaltbar sind. Dies erlaubt eine optimale Schonung säurelabiler Schutzgruppen z.B. an trifunktionellen Aminosäuren, insbesondere z.B. bei Synthesen mit repetitiven Kupplungs- und Deblockierungsschritten (Festphasen- oder Liquidphasentechnik). Ebenso erfahren z.B. tryptophanhlatige Peptide bei Desacylierungen in stärker saurem Milieu oft eine Zerstörung des Indolsystems, was sich in einer Violett-Verfärbung und UV-Abs.-Verschiebung äußert. Nebenreaktionen solcher Art können bei Verwendung milder Deblockierungsreagentien auf ein Minimum reduziert werden.

Im folgenden werden als Abkürzungen verwendet:

| | |
|---|---|
| Adoc | adamant-1-yl-oxycarbonyl |
| Boc | tert-Butyloxycarbonyl |
| BPCO | 2-Biphenylyl-4-propyl-2-oxycarbonyl |
| Trp | Tryptophan |
| $PCl_3$ | Phosphortrichlorid |
| $PCl_5$ | Phosphorpentachlorid |
| $POCl_3$ | Phosphoroxichlorid |
| TRH | Thyreotropin-freisetzendes Hormon |
| LH-RH | Leuteinisierendes Hormon-freisetzendes Hormon |
| FSH-RH | Follikel-stimulierendes Hormo-freisetzendes Hormon |
| Adpoc-OPh | (1-(1-Adamantyl)-1-methylethyl)-phenylcarbonat |
| Ad | Adamantyl |
| Gly | Glycin |
| Glu | Glutamin |
| Ala | Alanin |
| OSu | Hydroxysuccinimid |
| Lys | Lysin |
| Z | Benzyloxycarbonyl |
| Thr | Threonin |
| Bzl | Benzyl |
| Ph | Phenyl |
| Phe | Phenylalanin |
| Ser | Serin |
| OH | Hydroxy |
| $OCH_3$ | Methylester |
| Trt | Trityl |
| Cys | Cystein |
| DCC | Dicyclohexylcarbodiimid |
| DCHA | Dicyclohexylamin |
| DMF | Dimethylformamid |
| HOBt | 1-Hydroxybenzotriazol |
| HoSu | N-Hydroxysuccinimid |
| Triton B | Trimethylbenzylammoniumhydroxid |

Die vor kurzer Zeit entwickelten Schutzgruppen Bpoc, Ddz, Ppoc, Azoc (Abkürzungen nach den allgemeinen Regeln der Peptidchemic vgl. Houben-Weyl, Methoden der Organischen Chemie15/1, Georg Thieme Verlag Stuttgart 1974 und H. D. Jakubke und H. Jeschkeit, Aminosäuren-Peptide-Proteine, 2. Auflage, Akademie-Verlag Berlin 1973) tragen diesen Forderungen Rechnung, jedoch konnten nur die ersten beiden breitere Anwendung finden. In beiden Fällen ist die Lagerung der geschützten Aminosäuren aus Gründen autokatalytischer Zersetzung bzw. Photolabilität nicht ganz unproblematisch. Auch das zur Einführung der Bpoc-Gruppe oft benutzte Bpoc-Phenylcarbonat neigt

selbst bei tiefen Temperaturen zur Zersetzung. Eine Darstellung von Bpoc-Trp-OH gelingt nur mit sehr geringen Ausbeuten.

In der Literaturstelle E. Wünsch (ed.): Synthese von Peptiden. Methoden der organischen Chemie (Houben-Weyl), 4. Auflage, Band XV/1, Stuttgart (1974), Seiten 139 bis 141 sowie in der GB—A—1 106 272 werden Substanzen beschrieben, die eine sehr ähnliche Konstitution wie die beanspruchten Verbindungen aufweisen und ebenfalls als Schutzgruppenreagens Verwendung finden, beispielsweise bei Peptidsynthesen. Es handelt sich dabei um Adamant-1-yl-oxycarbonyl-chlorid (Adoc-Cl) und Derivate davon. Die anmeldungsgemäßen Verbindungen unterscheiden sich im wesentlichen nur durch eine eingeschobene Alkylengruppe der Formel —$C(R^1)(R^2)$—.

Bei der in der vorliegenden Anmeldung beanspruchten Verbindung handelt es sich um:

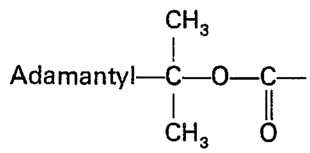

Bei den in der Literatur bekannten Verbindungen handelt es sich um die Adoc-Gruppe der folgenden Formel:

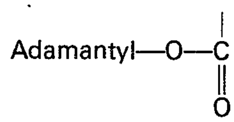

Der Fachmann hätte erwarten müssen, daß sich beide Gruppen ähnlich verhalten und gleiche oder ähnliche Reaktionsfähigkeiten aufweisen. Dies ist jedoch nicht der Fall. Es wurde überraschenderweise gefunden, daß sich die Adpoc-Gruppe wesentlich anders als die bekannte Adoc-Gruppe verhält. Im Gegensatz zur Adoc-Gruppe besitzt die Adpoc-Gruppe ein sterisch sehr stark gehindertes Kohlenstoffatom:

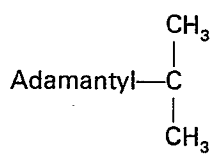

Durch das sterisch gehinderte Kohlenstoffatom wird erreicht, daß diese Schutzgruppe, bedingt durch die sterische Behinderung sehr schonend abgespalten wird. Die Abspaltung erfolgt also in wesentlich schwächer saurem Medium als dies für die Adoc-Gruppe der Fall ist. So konnte beispielsweise eindeutig nachgewiesen werden, daß sie die Schutzgruppe um den Faktor 1000 rascher abspalten läßt wie die bekannte Boc-Gruppe, welche die folgende Struktur aufweist:

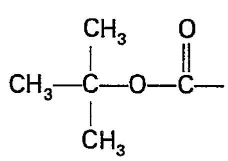

Die Boc-Gruppe ist wahrscheinlich die derzeit am haufigsten verwendete Schutzgruppe. Da sich die Boc-Gruppe von der Aminofunktion jedoch 1000 mal langsamer abspalten läßt als die erfindungsgemäße Adpoc-Gruppe, kann man sich vorstellen, daß die Seitenkettenschutzgruppen, welche während der Peptidsynthese intakt bleiben sollen, bei der Boc-Gruppenabspaltung auch angegriffen werden. Diese Nachteile besitzt die erfindungsgemäße Adpoc-Gruppe nicht. Es war überraschend und hat nicht nahegelegen, daß sich die erfindungsgemäße Adpoc-Gruppe gegenüber der bekannten Boc-Gruppe so stark unterscheidet. Die Adoc-Gruppe verhält sich ähnlich wie die Boc-Gruppe und somit sind bei allen Synthesen, bei denen die Adoc-Gruppe verwendet wird, dieselben Nachteile in Kauf zu nehmen, wie bei Synthesen unter Verwendung der Boc-Gruppe.

Bei Peptidsynthesen leidet bei der sauren Abspaltung besonders der Indolrest des Tryptophans und wird teilweise abgebaut und zerstört. Die Adpoc-Gruppe eignet sich daher besonders zur Darstellung von Peptidsequenzen mit Tryptophanrest. Die Anmelder konnten z.B. durch Lichtabsorptionsspektroskopie zeigen, daß bei der Abspaltung der Adpoc-Gruppe der Indolrest des Tryptophans überhaupt nicht zerstört wird.

Ein weiterer großer Vorteil der Adpoc-Gruppe ist, daß sie bsonders bei großen Peptiden stark solubilisierende Eigenschaften im Vergleich zu anderen Schutzgruppen also auch gegenüber der Adoc-Gruppe hat. Bestimmte Peptidsequenzen lassen sich so nur mit Hilfe der Adpoc-Gruppe in vernünftigen Mengen in Lösung bringen.

Die BPOC-Gruppe läßt sich im Vergleich zu der erfindungsgemäßen Adpoc-Gruppe um den Faktor 2—3 rascher abspalten. Man könnte meinen, dies sei vorteilhaft. Doch ist durch diese größere Labilität die Verbindung bei Lagerung nicht ausreichend stabil. Die BPOC-Gruppe ist weiterhin photolabil, was nicht unproblematisch ist, wie es von verschiedenen Forschergruppen, die sich mit Peptidsynthesen befassen, bekannt wurde. Insofern ist dieser äußerlich gering erscheinende Unterschied zwischen der erfindungsgemäßen Adpoc-Gruppe und der bekannten BPOC-Gruppe für die Praxis sehr entscheidend. Es war in keiner Weise vorherzusehen und ist völlig überraschend, daß sich die Adpoc-Gruppe einerseits unter so milden Bedingungen abspalten läßt, andererseits die Ausgangsmaterialien, also die sogenannten Adpoc-Aminosäuren, monatelang bie Zimmertemperatur haltbar sind. Die BPOC-Reagenzien sind oft leicht zersetzbar, was für die Adpoc-Aminosäuren in keiner Weise zutrifft. Wie R. S. Feinberg und R. B. Merrifield in Tetrahedron *28* 5865 (1972) publiziert haben, neigt das zur Einführung der BPOC-Gruppe oft benützte BPOC-Phenylkarbonat selbst bei tiefen Temperaturen zu Zersetzung. Dies gilt überraschenderweise für das Adpoc-Phenylkarbonat nicht. Das Adpoc-Phenylkarbonat ist sogar bei Zimmertemperatur stabil. Die Synthese von BPOC-Trp-OH gelingt nur mit geringen Ausbeuten. Wie die Anmelder in der Angewandten Chemie (H. Kalbacher und W. Voelter, Angew. Chemie 90, 998 (1978)) beschrieben haben, kann Adpoc-Trp-OH in 72%iger Ausbeute sehr gut hergestellt werden.

Überraschenderweise wurde nun jetzt gefunden, daß mit Hilfe der erfindungsgemäßen Verbindungen neue Schutzgruppen für Verbindungen mit primären und sekundären Aminofunktionen, insbesondere Aminosäuren, Aminozucker, biogene Amino, z.B. Tyramin, Dopamin, Noradrenalin, Adrenalin, Tryptamin, Serotonin, Melatonin, Histamin, Cysteamin, $\beta$-Alanin, $\gamma$-Amonobuttersäure, Propanolamin, Äthanolamin, Cadaverin, Purescin und Agmatin, eingeführt werden können, die folgende Vorteile gegenüber dem Stand der Technik aufweisen:

1. Die Einführung der Schutzgruppe gelingt mit Hilfe stabilier kirstalliner und reaktiver Verbindungen, wie dem Phenylcarbonat (Adpoc-OPh) oder dem Fluorameisensäureester (Adpoc-F).

2. Die Adpoc-Gruppe kann unter sehr milden Bedingungen entfert werden. Schutzgruppen vom t-Butyltyp bleiben unangetastet. Tryptophanhaltige Peptide bleiben unter Abspaltungsbedingungen stabil.

3. Die Schutzgruppe ist sowohl gegen Alkali, als auch gegenüber Hydrogenolyse stabil.

4. Aufgrund der ausgeprägten Lipophilie besitzt der Adpoc-Rest, insbesondere bei höheren Peptiden, stark lösungsvermittelnde Eigenschaften.

5. Adpoc-Trp-OH ist in hohen Ausbeuten darstellbar. Die Lagerung dieser Verbindungen bei normalen Lichtverhältnissen und bei Raumtemperatur führte nach 3 Monaten zu keiner Veränderung des Derivats.

6. Die in der Peptidchemie üblichen Methoden (DCCI/HOBt-Aktivesterkupplungen, Verseifungen, Hydrierungen) können unter Erhaltung des Adpoc-Restes angewandt werden.

Als besonders vorteilhaft erweist sich die Schutzgruppe 2-Adamantan(1)-propyl-2-oxycarbonyl, im folgenden Adpoc genannt, auch dadurch, daß die Aufhebung der N-[2-Adamantan-(1)]-isopropyl-Maskierung im milden acidolytischen Medium gelingt und zwar vorzugsweise in 3%iger Trifluoressigsäure in Methylenchlorid innerhalb 4 bis 5 Minuten bei 0°C oder innerhalb 2 bis 3 Minuten bei Raumtemperatur. Hierbei wird eine weitgehende Demaskierungsselektivität gegenüber Schutzgruppen vom tert.-Butyltyp erreicht. Selbstverständlich bleiben N-Benzyloxycarbonyl-, O-Benzyläther sowie -Ester-Gruppierungen unangetastet.

Die genannten Spaltungsbedingungen erlauben eine um den Faktor $10^3$ schnellere Abspaltung dieser neuen N-Urethanschutzgruppe gegenüber der N-tert.-Butyloxycarbonylgruppe. Gegenüber der N-[2-Biphenyl-(4)]-oxycarbonyl-(Bpoc)-Maskierung weisen Adpoc-Aminosäuren unter acidolytischen Bedingungen dagegen etwa 2- bis 3-fach höhere Stabilität auf.

Gegenstand der Erfindung sind verbindungen der allgemeinen Formel I

$$\mathrm{Ad{-}\overset{\displaystyle R^1}{\underset{\displaystyle R^2}{\overset{|}{\underset{|}{C}}}}{-}O{-}CO{-}X} \qquad (I)$$

in der Ad den 1-Adamantylrest, der substituiert durch Alkyl- mit 1—8 C-Atomen, Alkoxy- mit 1—8 C-Atomen, Nitrogruppen oder Halogen oder unsubstituiert sein kann, die Reste $R^1$ und $R^2$ gesättigte Al-

kylreste mit 1 bis 8 Kohlenstoffatomen, vorzugsweise Methylgruppen sind und X = Chlor, Fluor, Brom, Azido, Phenoxy, das gegebenenfalls im Benzolkern durch Fluor, Chlor, Brom, Jod, Nitro-, Alkoxy- oder Alkylgruppen substituiert sein kann, N-Oxysuccinimido oder Reste der Formeln

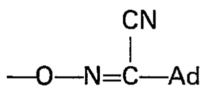

oder

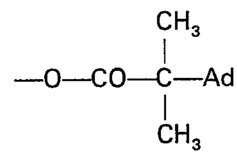

bedeuten.

Bevorzugte Verbindungen sind solche der folgenden Formel Ia

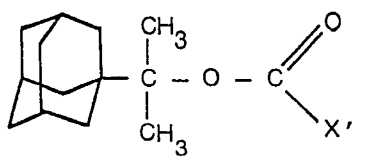

(Ia)

in der X' = Fluor, Chlor, Brom, Azido oder Phenoxy bedeuten sowie Verbindungen der allgemeinen Formel III und IV

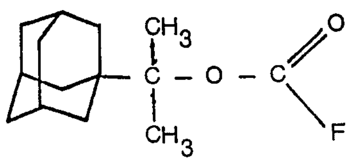

(III)

und

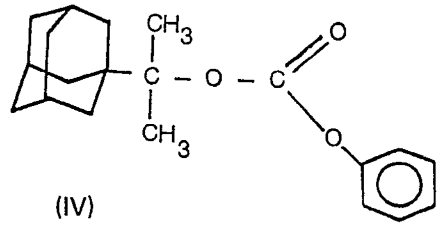

(IV)

Die Erfindung betrifft weiterhin ein Verfahren zur Herstellung der neuen Verbindungen der allgemeinen Formel I

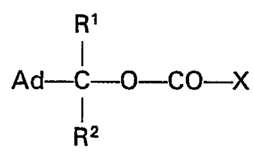

(I)

in der Ad den 1-Adamantylrest, der substituiert durch Alkyl- mit 1—8 C-Atomen, Alkoxy- mit 1—8 C-Atomen, Nitrogruppen oder Halogen oder unsubstituiert sein kann, die Reste $R^1$ und $R^2$ gesättigte Alkylreste mit 1 bis 8 Kohlenstoffatomen und X = Chlor, Brom, Fluor, Azido, Phenoxy, das gegebenenfalls im Benzolkern durch Fluor, Chlor, Brom, Jod, Nitro- oder Alkyl- oder Alkoxygruppen substituiert sein kann, N-Oxysuccinimido oder Reste der Formel

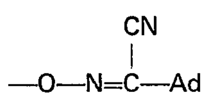

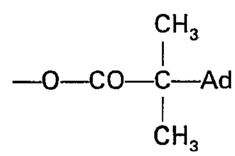

bedeuten, das dadurch gekennzeichnet ist, daß man die entsprechenden 1-Adamantancarbonsäuren mit geeigneten Phosphorhalogeniden oder Thionylchlorid behandelt, das erhaltene Reaktionsprodukt mit Alkoholen zu den 1-Adamantanniederalkylestern umsetzt und diese Verbindungen mit entsprechenden Alkylmagnesiumhalogeniden zu Verbindungen der Formel II

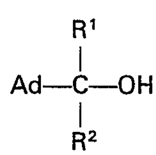

(II)

umsetzt, worin Ad, $R^1$ und $R^2$ die vorstehend angegebenen Bedeutungen haben und die Verbindungen der Formel II mit Verbindungen der allgemeinen Formel

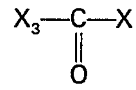

worin $X_3$, ein Halogenatom oder die Phenoxygruppe bedeutet und X die oben gegebene Bedeutung besitzt, umsetzt.

Gemäß einer bevorzugten Ausführungsform setzt man zur Herstellungf der Verbindungen der allgemeinen Formel Ia 1-Adamantancarbonsäure zuerst mit Phosphorpentachlorid/Äthanol um und setzt dann das erhaltene Reaktionsprodukt mit Methylmagnesiumjodid zu 2-Adamantan(1)-propanol(2) und $CO(X')_2$ um, worin X' die oben angegebenen Bedeutung besitzt.

Die Erfindung betrifft weiterhin die Verwendung der neuen Verbindungen der Formel I zur Herstellung von Verbindungen mit Peptidbindungen, insbesondere zur Herstellung von Verbindungen der allgemeinen Formel Ib

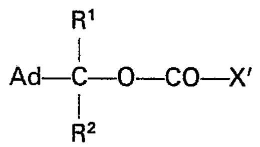

(1b)

in der Ad den 1-Adamantylrest, der substituiert durch Alkyl- mit 1—8 C-Atomen, Alkoxy- mit 1—8 C-Atomen, Nitrogruppen oder Halogen oder unsubstituiert sein kann, die Reste $R^1$ und $R^2$ gesättigte Alkylreste mit 1 bis 8 Kohlenstoffatomen und X' Reste primärer oder sekundärer Aminoverbindungen sind.

Bevorzugt werden die neuen Verbindungen der Formel I zur Darstellung von TRH, LH/FSH-RH, Eledoisin, Secretin, Gastrin, Physalaemin, Glucagon, Adrenocorticotropin, Oxytocin, Vasopressin, Calcitonin, Angiotensin, Bradikinin, Kalidin, Phyllokinin, Gramicidin, Bacitracin, Peptidantibiotika, Peptidtoxine, Peptidinsektizide, Somatostatin und jeweils deren Teilsequenzen sowie von Adpoc-Gly-OH, Adpoc-L-Ala-DCHA, Adpoc-L-Gln-OH, Adpoc-L-Trp-OH, Adpoc-L-Trp-OSu, Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-$OCH_3$, Adpoc-L-Trp-L-Lys(Z)-L-Thr-(Bzl)-L-Phe-L-Thr(Bzl)-L-Ser(OBzl)-$OCH_3$, sowie Adpoc-L-Trp-L-Lys(Z)-L-Thr(Bzl)-L-Phe-L-Thr(Bzl)-L-Ser(OBzl)-L-Cys(Trt)-OH verwendet.

Gemäß einer weiteren bevorzugten Auführungsform werden die neuen Verbindungen der Formel I zur Darstellung von Adpoc-L-Trp-OH, Adpoc-L-Trp-OSu, Adpoc-L-Trp-L-Lys(Z)-OH, Adpoc-L-Trp-L-Lys(Z)-L-Thr(Bzl)-L-Phe-L-Thr(Bzl)-L-Ser(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH, sowie Corticotropine, Melanotropine, LH/FSH-RH, Glucagon, Calcitonine, Gastrine, Somatostatin und Teilsequenzen als tryptophanhaltige Peptide verwendet.

Gemäß einer weiteren bevorzugten Ausführungsform werden die neuen Verbindungen der Formel I zur Darstellung von Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-L-Phe-OH, Adpoc-L-Thr(Bzl)-L-Phe-L-Thr(Bzl)-L-Ser(Bzl)-$OCH_3$, Adpoc-L-Trp-L-Lys(Z)-L-Thr(Bzl)-L-Phe-L-Thr(Bzl)-L-Ser-(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH sowie TRH, LH/FSH-RH, Eledoisin, Secretin, Gastrin, Physalaemin, Glucagon, Adrenocorticotropin, Oxytocin, Vasopressin, Calcitonin, Angiotensin, Bradikinin, Kalidin, Phyllokinin, Gramicidin, Bacitracin, Pentidantibiotika, Peptidtoxine, Peptidin-sektizide, Somatostatin und jeweils deren Teilsequenzen, die dadurch gekennzeichnet sind, daß die Aminosäuren mit den Verbindungen der Patentansprüche 1—3 und Benzyltrimethylammoniumhydroxide verwendet.

In den obigen Formeln bedeutet Halogen Fluor, Chlor, Brom oder Jod.

Die Herstellung der genannten 1-Adamantancarbonsäureester erfolgt aus den Säuren auf an sich bekannte Weise, z.B. aus den entsprechenden Säuren durch Behandlung mit geeigneten Phosphorhalogeniden und Umsetzung mit Alkoholen.

Unter Niederalkyl sind Alkylreste mit bis zu acht Kohlenstoffatomen zu verstehen.

Die nachstehenden Reaktionsschemata dienen zur Erläuterung des erfindungsgemäßen Verfahrens.

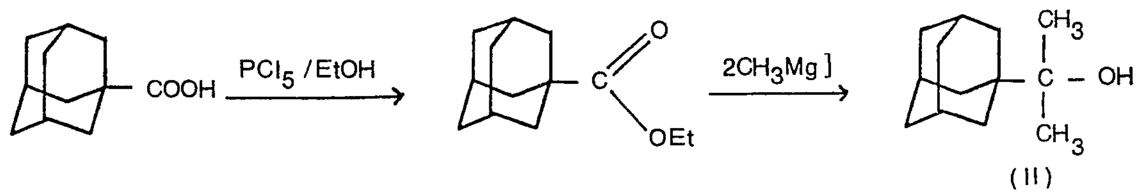


(II)

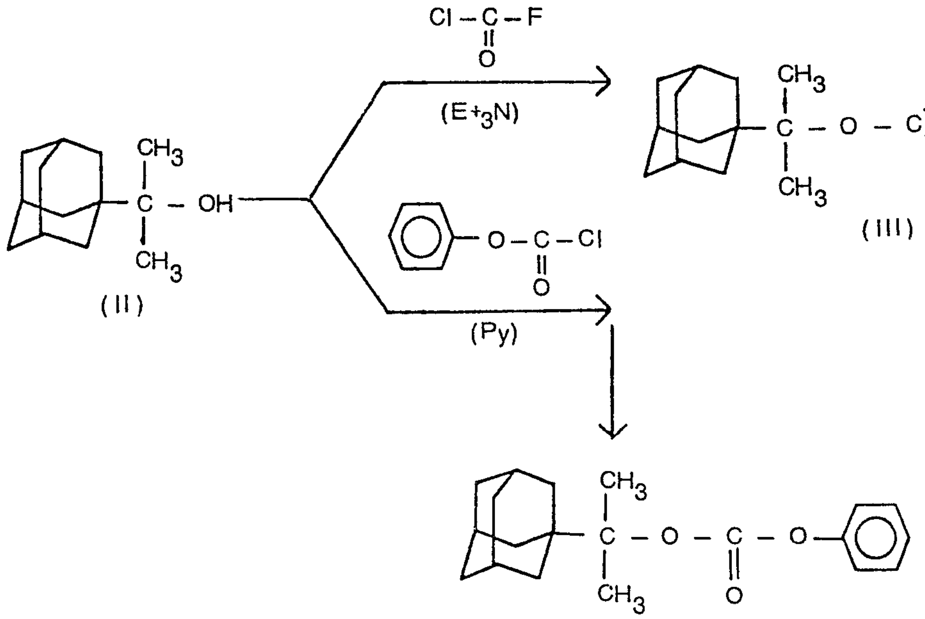


(II) (III) (IV)

Unter Aminosäuren sind zu verstehen organische Verbindungen mit einer Carboxylgruppe, welche zusätzlich noch eine $—NH_2—$ oder NH-Funktion besitzen, insbesondere aber Glycin, Alanin, Leucin, Isoleucin, Serin, Threonin, Cystein, Cystin, Methionin, Prolin, Hydroxyprolin, Lysin, Hydroxylysin, Arginin, Histidin, Asparaginsäure, Asparagin, Glutaminsäure, Glutamin, Phenylalanin, Adrenalin, Tryptamin, Serotonin, Melatonin, Histamin, Cysteamin, Propanolamin, Äthanolamin, Cadaverin, Putrescin und Agmatin. Unter Aminozucker sind zu verstehen Kohlenhydrate, monomeren oder polymerer Art, welche primäre ($—NH_2$) oder sekundäre (>NH—) Funktion besitzen, z.B. Glucosamin (= 2-Amino-2-desoxyglucose) oder Galactosamin (2-Amino-2-desoxygalactose).

Die erfindungsgemäßen Verbindungen eignen sich ausgezeichnet für Peptidsynthesen, insbesondere zur Synthese von TRH, LH/FSH-RH, Eledoisin, Secretin, Gastrin, Physalaemin, Glucagon, Adrenocorticotropin, Oxytocin, Vasopressin und Somatostatin.

Die nachfolgenden Beispiele sollen die erfindung näher erläutern, ohne sie einzuschränker.

Beispiele:

Die Schmelzpunkte wurden im Apparat nach Dr. Tottoli bestimmt und sind nicht korrigiert. Die spezifischen Drehwerte wurden mit einem Digitalpolarimeter OLD 5 der Fa. Zeiss ermittelt. Die Dünnschichtplatten (DE-Fertigplatten, Kieselgel $60F_{254}$) wurden von der Fa. Merck bezogen. Indikatoren: UV bei 254 nm Ninhydrin- und Chlortolidin-Reagens. Für die Dünnschichtchromatographie kamen folgende Fließmittelsysteme zur Anwendung:

A: Chloroform/Methanol/Benzol/$H_2O$ (40:40:40:5)
B: Butanol/Eisessig/Wasser (3:1:1)
C: Methanol/Chloroform (2:1)

Beispeil 1

A: Fluorameisensäure-2-adamantyl(1)-propyl(2)-ester-(Adpoc-F) (III)

Zu 19,4 g (0,1 Mol) 2-Adamantan(1)-propanol(2), hergestellt nach Tetrahedron *27*, 3 (1971), in 150 ml trockenem Äther und 14 ml Triäthylamin wird im Laufe von 2—3 Stunden trckenes, $SO_3$-freies Carbonylchloridfluorid (aus 60 g 65%igen Oleum und 25 ml Fluorotrichloromethan gewonnen) bei —40° direkt einkondensiert. Hierbei fällt Triäthylaminhydrochlorid aus. Nach Beendigung der Gasentwichlung beläßt man das Gemisch über Nacht bei —20°, danach wird bei 200 Torr und 10° entgast. Nach Filtration wird der Rückstand auf dem Filter mit trockenem Äther nachgewaschen. Das Filtrat enthält 22,9 g Adpoc-F. Es kann sowohl in ätherischer Lösung oder direkt nach Einengen im Vakuum

eingesetzt werden. Nach Entfernen des Lösungsmittels im Vakuum kann der Fluorameisensäureester in kristalliner Form gewonnen werden. Bei dieser Umsetzungsmethode kann IR-spektroskopisch kein Carbinol mehr nachgewiesen werden.

Fp. 52°C.
IR (KBr) 1815 $cm^{-1}$
Ausbeute: 22,9 g (95%)
$C_{14}H_{21}O_2F$ (240.32)

| | | |
|---|---|---|
| Ber. | C 69,9 | H 8,81 |
| Gef. | C 70.25 | H 8.99 |

B. Herstellung der Adpoc-Aminosäuren

10 mMol der Aminosäure werden unter Erwärmen in 4,5 $cm^3$ ca. 40%iger Lösung von Triton-B* in Methanol gelöst. Man dampft im Vakuum ein, nimmt den Rückstand in 5—10 $cm^{-3}$ Dimethylformamid auf, dampft wiederum ein und wiederholt den Vorgang. Nun löst man in 10 $cm^3$ Dimethylformamid, kühlt auf —10° ab und versetzt bei dieser Temperatur mit 2,65 g (11 mMol) Adpoc-F und 1,6 $cm^3$ Triäthylamin. Man rührt 3 Stunden bei —5°C, 2 Stunden bei 0°C, läßt auf Raumtemperatur kommen und gießt die Reaktionslösung in 20 $cm^3$ Eiswasser. Nach 2-facher Extraktion mit je 20 ml Äther-Petroläther (1:1) wird die wäßrige Phase mit Zitronensäure angesäuert und mehrfach mit insgesamt 80 $cm^3$ Äther extrahiert. Die vereinigten Ätherauszüge werden mit Wasser neutral gewaschen; der Äther wird nach kurzem Trocknen über $Na_2SO_4$ im Vakuum abdestilliert. Der Rückstand wird entweder direkt oder nach Überführen in das DCHA-Salz zur Kristallisation gebracht. Die Eigenschaften von Adpoc-Aminosäuren, die nach diesem Verfahren hergestellt wurden, sind in Tabelle 1 aufgeführt.

## Beispiel 2

A. [2-Adamantan(1)-propyl(2)]phenyl-carbonat (IV)

19,4 g (0,1 Mol) 2-Adamantan(1)propanol(2), 100 ml Dichlormethan und 12.0 ml Pyridin werden bei —5°C innerhalb 30 Minuten mit einer Lösung von 15,2 ml Chlorameisensäurephenylester in 50 ml Dichlormethan versetzt. Die während des Zutropfens gebildete Fällung geht nach Rühren über Nacht bei 0°C wieder in Lösung. Man gießt das Reaktionsgemisch auf Eis, verdünnt mit 150 ml Dichlormethan und trennt die organische Phase ab. Nach 3-maligem Waschen mit Wasser wird über Natriumsulfat getrocknet und bei 30° im Vakuum eingedampft. Durch 2-malige Umkristallisation aus Benzol/n-Hexan werden lange farblose Kristalle erhalten.

Fp.: 72°C
Ausbeute: 26,25 g (83,5%)
$C_{20}H_{26}O_3$ (314.43)

| | | |
|---|---|---|
| Ber. | C 76,40 | H 8,33 |
| Gef. | C 76,55 | H 8,43 |

B. Herstellung der Adpoc-Aminosäuren

10 mMol der Aminosäre werden unter gelindem Erwärmen in 4,6 $cm^3$ einer ca. 40%igen Lösung von Triton B* in Methanol gelöst. Nach Verdampfen des Lösungsmittels wird der Rückstand durch 2-malige azeotrope Destillation mit je 30 ml Dimethylformamid vom Wasser befreit. Der Rückstand wird bei 50°C und 30 ml Dimethylformamid aufgenommen, mit 3,8 g (12 mMol) Adpoc-OPh versetzt und die Reaktionsmischung 3 Stunden bei 50°C gerührt. Zur Aufarbeitung wird zwischen Wasser und Diäthyläther-Petroläther (1:1) verteilt, die abgetrennte wäßrige Phase bei 0.2C mit 1 N Zitronensäurelösung angesäuert (pH 2—3) und mit Diäthyläther erschöpfend extrahiert. Nach üblichem Waschen und Trocknen der Ätherauszüge wird im Vakuum zur Trockene eingedampft. Der verbleibende Rückstand wird entweder direkt oder nach Überführen in das DCHA-Salz zur Kristallisation gebracht.

Die Eigenschaften von Adpoc-Aminosäuren, die nach diesem Verfahren hergestellt werden, sind in Tabelle 1 aufgeführt.

Triton B = Benzyltrimethylammoniumhydroxid

TABELLE 1

Herstellung und Eigenschaften von Adpoc Aminosäuren und Peptiden+)

| Aminosaurederivat und Elementarformel | Mol. Gew. | Ausb. % | Fp(°C) | $[\alpha]_D^{20}$ | Analyse Ber. Gef. C% | H% | N% | DC $Rf_A$ |
|---|---|---|---|---|---|---|---|---|
| Adpoc–Gly–OH $C_{16}H_{25}N_{04}$ | 295.38 | 74 | 135°C | – | 65.06 64.95 | 8.53 8.72 | 4.74 4.81 | 0.37 |
| Adpoc–Gly–DCHA $C_{28}H_{48}N_2O_4$ | 476.70 | | 158°C | – | 70.54 70.69 | 10.15 10.20 | 5.88 5.89 | |
| Adpoc–Ala.DCHA $C_{29}H_{50}N_2O_4$ | 490.73 | 65 | 155°C | +6.22° (c=0.65, EtOH) | 70.98 71.20 | 10.27 10.17 | 5.70 5.79 | 0.45 |
| Adpoc–Gln–OH $C_{19}H_{30}N_2O_5$ | 366.46 | 61 | 98°C | –2.96° (c=0.8, EtOH) | 62.27 62.30 | 8.25 8.19 | 7.64 7.58 | 0.38 |
| Adpoc–Trp–OH $C_{25}H_{32}N_2O_4$ | 424.54 | 82 | 116° | –6.8° (c=1.04 MeOH) | 70.73 | 7.60 | 6.60 | 0.48 |
| Adpoc–Trp–OSu $C_{29}H_{35}N_3O_6$ | 521.62 | 92 | 67–69° (Zers.) | –19.6 (c=0.98, DMF) | 66.78 | 6.76 | 8.05 | 0.64 |
| Adpoc–Trp–Lys(Z)–OH $C_{39}H_{50}N_4O_7$ | 686.86 | 73 | 114–116° | –8.7° (c=0.5 MeOH) | 68.20 69.32 | 7.33 7.45 | 8.16 7.97 | 0.47 |

+) mit Ausnahme vo Glycin handelt es sich um L-Aminosäurederivative

TABELLE 1 (Fortsetzung)

| Aminosäurederivat und Elementarformel | Mol. Gew. | Ausb. % | Fp(°C) | $[\alpha]_D^{20}$ | Analyse Ber. Gef. C% | H% | N% | DC $Rf_A$ |
|---|---|---|---|---|---|---|---|---|
| Adpoc–Thr(Bzl)–OH $C_{25}H_{35}NO_5$ | 429.56 | 69 | 50–52° | +19.8° (c=10.2, MeOH) | 69.90<br>70.12 | 8.21<br>8.30 | 3.26<br>3.24 | |
| Adpoc–Thr(Bzl)–OH.DCHA $C_{37}H_{38}N_2O_5$ | 610.88 | | 130–131° | +12.9° (c=1.03, MeOH) | 72.75<br>72.91 | 9.57<br>9.80 | 4.58<br>4.71 | 0.60 |
| Adpoc–Thr(Bzl)–Phe–OH $C_{34}H_{44}N_2O_6$ | 576.74 | 67 | 75–76° (Zers.) | –18.67° (c=0.7, DMF) | 70.81<br>70.99 | 7.69<br>7.75 | 4.86<br>4.73 | 0.53 |
| Adpoc–Thr(Bzl)–Phe–Thr(Bzl)–Ser(Bzl)–$OCH_3$ $C_{56}H_{70}N_4O_{10}$ | 959.20 | 73 | 81° | +19.65° (c=0.8, DMF) | 70.12<br>69.65<br>70.39 | 7.36<br>7.36<br>7.57 | 5.84<br>5.70<br>5.80 | 0.70 |
| Adpoc–Trp–Lys(Z)–Thr–(Bzl)–Phe–Thr(Bzl)–Ser–(Bzl)–$OCH_3$ $C_8H_{98}N_8O_{14}$ | 1407.73 | 68 | 164–165° | +11.7° (c=0.5, DMF) | 69.11<br>69.29 | 7.01<br>7.11 | 7.96<br>8.04 | 0.73 |
| Adpoc–Trp–Lys(Z)–Thr–(Bzl)–Phe–Thr(Bzl)–Ser–(Bzl)–Cys(Trt)OH $C_{102}H_{115}N_9O_{15}S$ | 1739.12 | 82.4 | 150–152° | +8.6° (c=0.45, DMF) | 70.44<br>70.31 | 6.66<br>6.58 | 7.24<br>7.36 | 0.76 |

+) mit Ausnahme vo Glycin handelt es sich um L-Aminosäurederivate

**Patentansprüche**

1. Verbindungen der allgemeinen Formel I

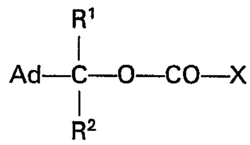

(I)

in der Ad den 1-Adamantylrest, der substituiert durch Alkyl- mit 1—8 C-Atomen, Alkoxy- mit 1—8 C-Atomen, Nitrogruppen oder Halogen oder unsubstituiert sein kann, die Reste $R^1$ und $R^2$ gesättigte Alkylreste mit 1 bis 8 Kohlenstoffatomen, vorzugsweise Methylgruppen sind und X = Halogen, Azido, Phenoxy, das gegebenenfalls im Benzolkern durch Fluor, Chlor, Brom, Jod, Nitro-, Alkoxy- oder Alkylgruppen substituiert sein kann, N-Oxysuccinimido oder Reste der Formeln

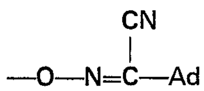

oder

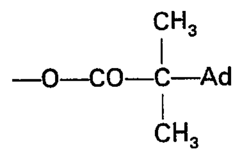

bedeuten.

2. Verbindungen nach Anspruch 1 der allgemeinen Formel Ia

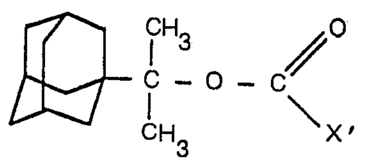

(Ia)

in der X' = Fluor, Chlor, Brom, Azido oder Phenoxy bedeutet.

3. Die Verbindungen nach Anspruch 1 der folgenden allgemeinen Formeln III und IV

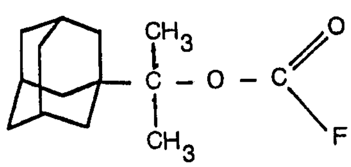

(III)

und

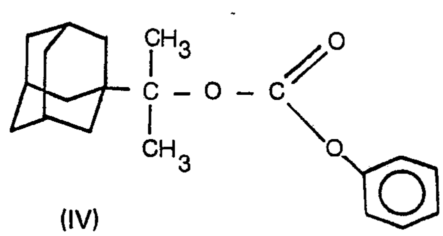

(IV)

4. Verfahren zur Herstellung der neuen Verbindungen der allgemeinen Formel I

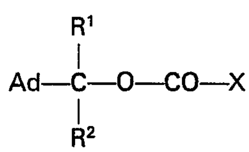

(I),

in der Ad den 1-Adamantylrest, der substituiert durch Alkyl- mit 1—8 C-Atomen, Alkoxy- mit 1—8 C-Atomen, Nitrogruppen oder Halogen oder unsubstituiert sein kann, die Reste $R^1$ und $R^2$ gesättigte Alkylreste mit 1 bis 8 Kohlenstoffatomen und X = Halogen, Azido, Phenoxy, das gegebenenfalls im Benzolkern durch Fluor, Chlor, Brom, Jod, Nitro- oder Alkyl- oder Alkoxygruppen substituiert sein kann, N-Oxysuccinimido oder Reste der Formeln

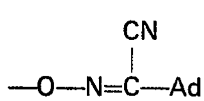

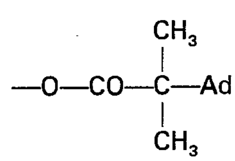

bedeuten, dadurch gekennzeichnet, daß man die entsprechenden 1-Adamantancarbonsäuren mit geeigneten Phosphorhalogeniden oder Thionylchlorid behandelt, das erhaltene Reaktionsprodukt mit Alkoholen zu den 1-Adamantanniederalkylestern umsetzt und diese Verbindungen mit entsprechenden Alkylmagnesiumhalogeniden zu Verbindungen der Formel II

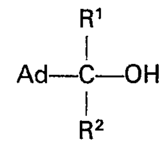

(II)

umsetzt, worin Ad, $R^1$ und $R^2$ die vorstehend angegebenen Bedeutungen haben, und die Verbindungen der Formel II mit Verbindungen der allgemeinen Formel

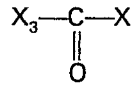

worin $X_3$ ein Halogenatom oder die Phenoxygruppe bedeutet und X die oben gegebene Bedeutung besitzt, umsetzt.

5. Verfahren zur Herstellung der Verbindungen der allgemeinen Formel Ia gemäß Anspruch 2, dadurch gekennzeichnet, daß man 1-Adamantancarbonsäure zuerst mit Phosphorpentachlorid/Äthanol umsetzt, das erhaltene Reaktionsprodukt dann mit Methylmagnesiumjodid zu 2-Adamantan(1)-propanol(2) umsetzt und dieses mit $CO(X')_2$ umsetzt, worin X' die in Anspruch 2 angegebene Bedeutung hat.

6. Verwendung der neuen Verbindungen der Formel I zur Herstellung von Verbindungen mit Peptidbindungen.

7. Verwendung der neuen Verbindungen gemäß Anspruch 6 zur Herstellung von Verbindungen der allgemeinen Formel Ib

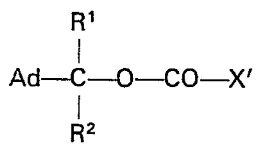

(Ib)

in der Ad den 1-Adamantylrest, der substituiert durch Alkyl- mit 1—8 C-Atomen, Alkoxy- mit 1—8 C-Atomen, Nitrogruppen oder Halogen oder unsubstituiert sein kann, die Reste $R^1$ und $R^2$ gesättigte Alkylreste mit 1 bis 8 Kohlenstoffatomen und X' Reste primärer oder sekundärer Aminoverbindungen sind.

8. Verwendung der neuen Verbindungen der Formel I gemäß Anspruch 6 zur Darstellung von TRH, LH/FSH-RH, Eledoisinen, Secretinen, Gastrinen, Physalaeminen, Glucagonen, Adrenocorticotropinen, Oxytocinen, Vasopressinen, Calcitoninen, Angiotensinen, Bradikininen, Kalidinen, Phyllokininen, Gramicidinen, Bacitracinen, Peptidantibiotika, Peptidtoxinen, Peptidinsektiziden, Somatostatinen und jeweils deren Teilsequenzen sowie von Adpoc-Gly-OH, Adpoc-L-Ala-DCHA, Adpoc-L-Gln-OH, Adpoc-L-Trp-OH, Adpoc-L-Trp-OSu, Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-$OCH_3$, Adpoc-L-Trp-L-Lys(Z)-L-Thr(Bzl)-L-Phe-L-Thr(Bzl)-L-Ser(OBzl)-$OCH_3$, sowie Adpoc-L-Lys(Z)-L-Thr(Bzl)-L-Phe-L-Thr-(Bzl)-L-Ser(OBzl)-L-Cys(Trt)-OH.

9. Verwendung ner neuen Verbindungen der Formel I gemäß Anspruch 6 zur Darstellung von Adpoc-L-Trp-OH, Adpoc-L-Trp-OSu, Adpoc-L-Trp-L-Lys(Z)-OH, Adpoc-L-Trp-L-Lys(Z)-L-Thr(Bzl)-L-Phe-L-Thr(Bzl)-L-Ser(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH, sowie Corticotropinen, Melanotropinen, LH/FSH-RH, Glucagonen, Calcitoninen, Gastrinen, Somatostatinen und Teilsequenzen als tryptophanhaltige Peptide.

10. Verwendung der neuen Verbindungen der Formel I gemäß Anspruch 6 zur Darstellung von Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-L-Phe-OH, Adpoc-L-Thr(Bzl)-L-Phe-L-Thr(Bzl)-L-Ser(Bzl)-$OCH_3$, Adpoc-L-Trp-L-Lys(Z)-L-Thr(Bzl)-L-Phe-L-Thr(Bzl)-L-Ser(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH sowie TRH, LH/FSH-RH, Eledoisinen, Secretinen, Gastrinen, Physalaeminen, Glucagonen, Adrenocorticotropinen, Oxytocinen, Vasopressinen, Calcitoninen, Angiotensinen, Bradikininen, Kalidinen, Phyllokininen, Gramicidininen, Bacitracinen, Peptidantibiotika, Peptidtoxinen, Peptidinsektiziden, Somatostatinen und jeweils deren Teilsequenzen, dadurch gekennzeichnet, daß die Aminosäuren mit den Verbindungen der Patentansprüche 1—3 und Benzyltrimethylammoniumhydroxid umgesetzt werden.

## Claims

1. Compounds corresponding to the following general formula

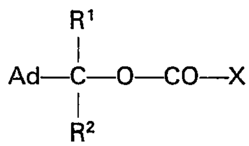

(I)

in which Ad represents the 1-adamantyl radical which may be substituted by $C_1$—$C_8$-alkyl, $C_1$—$C_8$-alkoxy, nitro groups or halogen or unsubstituted, the radicals $R^1$ and $R^2$ are saturated $C_1$—$C_8$-alkyl radicals, preferably methyl groups and X represents halogen, azido, phenoxy, which may optionally be substituted in the benzene nucleus by fluorine, chlorine, bromine, iodine, nitro, alkoxy or alkyl groups, N-oxysuccinimido or radicals corresponding to the formula

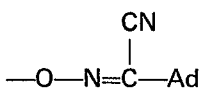

or

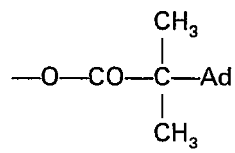

2. Compounds as claimed in Claim 1 corresponding to the following general formula

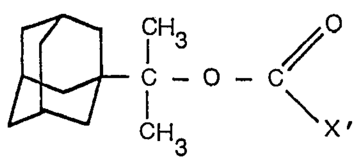

(Ia)

in which X' represents fluorine, chlorine, bromine, azido or phenoxy.

3. Compounds as claimed in Claim 1 corresponding to the following general formulae

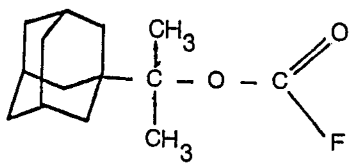

(III)

and

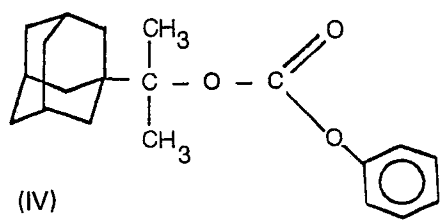

(IV)

4. A process for producing the new compounds corresponding to the following general formula

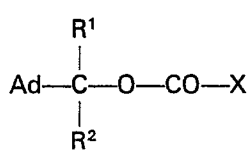

(I)

in which Ad represents the 1-adamantyl radical which may be substituted by $C_1$—$C_8$-alkyl, $C_1$—$C_8$-alkoxy, nitro groups or halogen or unsubstituted, the radicals $R^1$ and $R^2$ represent saturated $C_1$—$C_8$-alkyl radicals and X represents halogen, azido, phenoxy, which may optionally be substituted in the benzene nucleus by fluorine, chlorine, bromine, iodine, nitro or alkyl or alkoxy groups, N-oxysuccinimido or radicals corresponding to the formulae

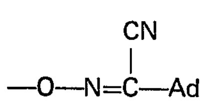

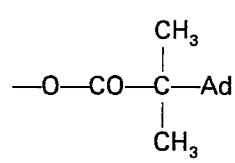

characterised in that the corresponding 1-adamantane carboxylic acids are treated with suitable phosphorus halides or with thionyl chloride, the reaction product obtained is reacted with alcohols to form the 1-adamantane lower alkyl esters and these compounds are reacted with corresponding alkyl

magnesium halides to form compounds corresponding to the following formula

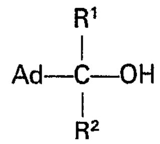 (II)

in which Ad, $R^1$ and $R^2$ are as defined above, and the compounds of formula (II) are reacted with compounds corresponding to the following general formula

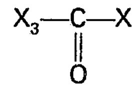

in which $X_3$ is a halogen atom or the phenoxy group and X is as defined above.

5. A process for producing the compounds corresponding to general formula (Ia) in Claim 2, characterised in that, first, 1-adamantane carboxylic acid is reacted with phosphorus pentachloride/ethanol, the reaction product obtained is then reacted with methyl magnesium iodide to form 2-adamantane(1)-propanol(2) and this compound is reacted with $CO(X')_2$ where X' has the meaning defined in Claim 2.

6. The use of the new compounds corresponding to formula I for the production of compounds containing peptide bonds.

7. The use of the new compounds claimed in Claim 6 for the production of compounds corresponding to the following general formula

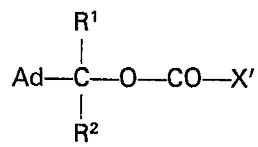 (Ib)

in which Ad represents the 1-adamantyl radical which may be substituted by $C_1$—$C_8$-alkyl, $C_1$—$C_8$-alkoxy, nitro groups or halogen or unsubstituted, the radicals $R^1$ and $R^2$ are saturated $C_1$—$C_8$-alkyl radicals and X' represents residues of primary or secondary amino compounds.

8. The use of the new compounds of formula (I) as claimed in Claim 6 for the preparation of TRH, LH/FSH-RH, eledoisins, secretins, gastrins, physalaemins, glucagons, adrenocorticotropins, oxytocins, vasopressins, calcitonins, angiotensins, bradikinins, kalidins, phyllokinins, gramicidins, bacitracins, peptide antibiotics, peptide toxins, peptide insecticides, somatostatins and partial sequences thereof and for the preparation of Adpoc-Gly-OH, Adpoc-L-Ala-DCHA, Adpoc-L-Gln-OH, Adpoc-L-Trp-OH, Adpoc-L-Trp-OSu, Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-$OCH_3$, Adpoc-L-Trp-L-Lys(Z)-L-Thr(Bzl)-L-Phe-L-Thr(Bzl)-L-Ser(OBzl)-$OCH_3$ and Adpoc-L-Trp-L-Lys(Z)-L-Thr(Bzl)-L-Phe-L-Thr(Bzl)-L-Ser(OBzl)-L-Cys(Trt)-OH.

9. The use of the new compounds of formula (I) as claimed in Claim 6 for the preparation of Adpoc-L-Trp-OH, Adpoc-L-Trp-OSu, Adpoc-L-Trp-L-Lys(Z)-OH, Adpoc-L-Trp-L-Lys(Z)-L-Thr(Bzl)-L-Phe-L-Thr(Bzl)-L-Ser(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH and of corticotropins, melanotropins, LH/FSH-RH, glucogons, calcitonins, gastrins, somatostatins and partial sequences thereof as tryptophane-containing peptides.

10. The use of the new compounds of formula (I) as claimed in Claim 6 for the preparation of Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-L-Phe-OH, Adpoc-L-Thr(Bzl)-L-Phe-L-Thr(Bzl)-L-Ser(Bzl)-$OCH_3$, Adpoc-L-Trp-L-Lys(Z)-L-Thr(Bzl)-L-Phe-L-Thr(Bzl)-L-Ser(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH and of TRH, LH/FSH-RH, eledoisins, secretins, gastrins, physalaemins, glucagons, adrenocorticotropins, oxytocins, vasopressins, calcitonins, angiotensins, bradikinins, kalidins, phyllokinins, gramicidins, bacitracins, peptide antibiotics, peptide toxins, peptide insecticides, somatostatins and partial sequences thereof, characterised in that the amino acids are reacted with the compounds claimed in Claims 1 to 3 and benzyl trimethyl ammonium hydroxide.

**Revendications**

1. Composés de formule générale I ci-après:

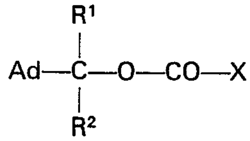

(I)

dans laquelle Ad représente le radical 1-adamantyle qui peut être substitué par des groupes alkyle comportant 1 à 8 atomes de carbone, des groupes alcoxy comportant 1 à 8 atomes de carbone, des groupes nitro — ou des atomes d'halogènes — ou être non substitué, les radicaux $R^1$ et $R^2$ représentent des radicaux alkyle saturés comportant 1 à 8 atomes de carbone, de préférence des groupes méthyle et X représente un atome d'halogène, un groupe azido, un groupe phénoxy qui peut être éventuellement substitué sur le noyau benzène par des atomes de fluor, de chlore, de brome, d'iode ou par des groupes introalcoxy ou alkyle, ou bien X représente un groupe N-oxysuccinimido- ou des radicaux répondant aux formules:

(a) 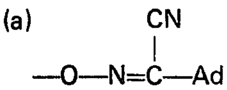 ou (b) 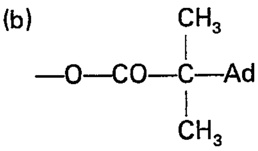

2. Composés selon la revendication 1, de formule générale Ia ci-après:

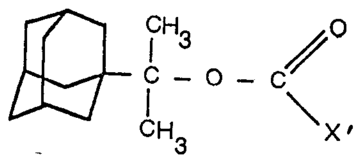

(Ia)

dans laquelle X' représente un atome de fluor, de chlore ou de brome ou un groupe azido ou phénoxy.

3. Composés selon la revendication 1, répondant aux formules générales III et IV ci-dessous

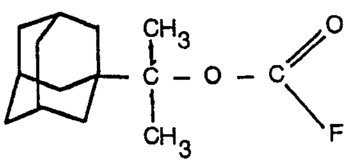

(III)

et

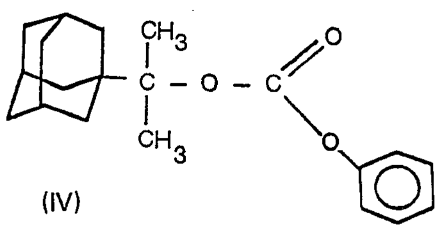

(IV)

4. Procédé de préparation des nouveaux composés de formule générale I ci-après:

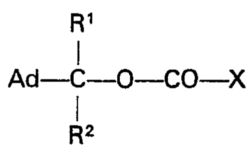

(I)

dans laquelle Ad représente le radical 1-adamantyle qui peut être substitué par des groupes alkyle comportant 1 à 8 atomes de carbone, des groupes alcoxy comportant 1 à 8 atomes de carbone, des groupes nitro ou des atomes d'halogènes ou peut être non substitué, les radicaux $R^1$ et $R^2$ représentent des radicaux alkyle saturés comportant 1 à 8 atomes de carbone et X représente un atome d'halogène, un groupe azido, un groupe phénoxy qui peut être éventuellement substitué sur le noyau benzène par des atomes de fluor, de chlore, de brome, d'iode ou par des groupes alkyle ou alcoxyl, ou bien X représente un groupe N-oxysuccinimido ou des radicaux répondant aux formules:

(a) 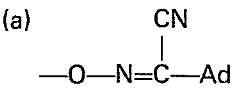 ou (b) 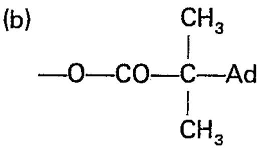

caractérisé en ce que l'on traite les acides 1-adamantanecarboxyliques correspondants par des halogénures de phosphore appropriés ou par le chlorure de thionyle, en ce que l'on fait réagir le produit de réaction obtenu avec des alcools pour obtenir des esters de 1-adamantane et d'alkyle inférieur et en ce que l'on fait réagir ces composés avec des halogénures d'alkylmagnésium correspondants pour obtenir des composés répondant à la formule II ci-après:

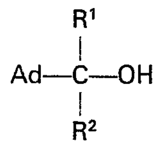

(II)

dans laquelle Ad, $R^1$ et $R^2$ ont les significations indiquées plus haut, et en ce que l'on fait réagir les composés de formule II avec des composés répondant à la formule générale ci-après:

(c) 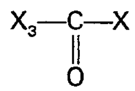

dans laquelle $X_3$ représente un atome d'halogène ou le groupe phénoxy et X a la signification donnée plus haut.

5. Procédé de préparation des composés de formule générale Ia selon la revendication 2, caractérisé en ce que l'on fait tout d'abord réagir l'acide 1-adamantanecarboxylique avec un mélange de pentachlorure de phosphore et d'éthanol, en ce que l'on fait ensuite réagir le produit de réaction obtenu avec l'iodure de méthylmagnésium pour obtenir le 2-adamantane(1)-propanol(2) et en ce que l'on fait réagir celui-ci avec un $CO(X')_2$ dans lequel X' a la signification indiquée dans la revendication 2.

6. Utilisation des nouveaux composés de la formule I pour la préparation de composés comportant des liaisons peptide.

7. Utilisation des nouveaux composés selon la revendication 6 pour la préparation de composés répondant à la formule générale Ib ci-après:

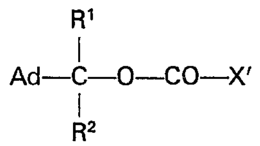

(Ib)

dans laquelle Ad représente le radical 1-adamantyle qui peut être substitué par des groupes alkyle comportant 1 à 8 atomes de carbone, des groupes alkoxy comportant 1 à 8 atomes de carbone, des groupes nitro, ou des atomes d'halogènes ou peut être non substitué, les radicaux $R^1$ et $R^2$ représentent des radicaux alkyle saturés comportant 1 à 8 atomes de carbone et X' représente des radicaux de composés aminés primaires ou secondaires.

8. Utilisation des nouveaux composés de formule I selon la revendication 6 pour la préparation de TRH, de LH/FSH-RH, d'élédoïsines, de sécrétines, de gastrines, de physalémines, de glucagons, d'adrénocorticotropines, d'oxytocines, de vasopressines, de calcitonines, d'angiotensines, de bradykinines, de kalidines, de phyllokinines, de gramicidines, de bacitracines, d'antibiotiques peptidiques, de toxines peptidiques, d'insecticides peptidiques, de somatostatines et des séquences partielles de chacun de ces corps, ainsi que de Adpoc-Gly-OH, Adpoc-L-Ala-DCHA, Adpoc-L-Gln-OH, Adpoc-L-Trp-OH, Adpoc-L-Trp-OSu, Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl-L-Ser-(Bzl)-$OCH_3$, Adpoc-L-Lys(Z)-L-Thr(Bzl)-L-Phe-L-Thr-(Bzl)-L-Ser(OBzl)-$OCH_3$ et Adpoc-L-Trp-L-Lys(Z)-L-Thr(Bzl)-L-Phe-L-Thr(Bzl)-L-Ser(OBzl)-L-Cys(Trt)-OH.

9. Utilisation des nouveaux composés de formule I selon la revendication 6 pour l'obtention de Adpoc-L-Trp-OH, Adpoc-L-Trp-OSu, Adpoc-L-Trp-L-Lys(Z)-OH, Adpoc-L-Trp-L-Lys(Z)-L-Trh(Bzl)-L-Phe-L-Thr(Bzl)-L-Ser(OBzl)-$OCH_3$, Adpoc-L-Lys(Z)-L-Thr(Bzl-L-Phe-L-Thr(Bzl-L-Ser(OBzl)-L-Cys(Trt)-OH, ainsi que de corticotropines, de mélanotropines, de LH/FSH-RH, de glucagons, de calcitonines, de gastrines, de somatostatines et de séquences partielles, sous forme de peptides contenant du tryptophane.

10. Utilisation des nouveaux composés de formule I selon la revendication 6 pour l'obtention de Adpoc-L-Trp-L-Lys(Z)-OH, Adpoc-L-Thr(Bzl)-OH, Adpoc-L-Thr(Bzl)-L-Phe-$OCH_3$, Adpoc-L-Thr(Bzl)-L-Phe-OH, Adpoc-L-Thr(Bzl)-L-Phe-L-Thr(Bzl)-L-Ser(Bzl)-L-Phe-OH, Adpoc-L-Thr(Bzl)-L-Phe-L-Thr-(Bzl)-L-Ser(Bzl)$OCH_3$, Adpoc-L-Trp-L-Lys(Z)-L-Thr(Bzl)-L-Phe-L-Thr(Bzl)-L-Ser(OBzl)-$OCH_3$, Adpoc-L-Trp-L-Lys(Z)-L-Thr(Bzl)-L-Phe-L-Thr(Bzl)-L-Ser(OBzl)-L-Cys(Trt)-OH ainsi que de TRH, LH/FSH-RH, d'élédoïsines, de sécrétines, de gastrines, de physalémines, de glucagons, d'acrénocorticotropines, d'oxytocines, de vasopressines, de calcitonines, d'angiotensines, de bradykinines, de kalidines, de phyllokinines, de gramicidines, de bacitracines, d'antibiotiques peptidiques, de toxines peptidiques, d'insecticides peptidiques, de somatostatines et des séquences partielles de chacun de ces corps, caractérisé par le fait que l'on fait réagir les aminoacides avec les composés selon les revendications 1 à 3 et avec l'hydroxyde de benzyl-triméthylammonium.